# EUROPEAN PATENT SPECIFICATION

(11) **EP 4 631 424 B1**
(45) Date of publication and mention of the grant of the patent: **18.11.2026**
(21) Application number: 23915015.4
(22) Date of filing: 28.12.2023
(51) Int. Cl.: A61B 5/024, A61B 5/00, A61B 5/0205, A61B 5/1455, G06V 10/141

(54) **METHOD FOR OBTAINING BIOMETRIC INFORMATION THROUGH CAMERA, AND ELECTRONIC DEVICE THEREFOR**
VERFAHREN ZUR GEWINNUNG BIOMETRISCHER INFORMATIONEN ÜBER EINE KAMERA UND ELEKTRONISCHE VORRICHTUNG DAFÜR
PROCÉDÉ D'OBTENTION D'INFORMATIONS BIOMÉTRIQUES PAR L'INTERMÉDIAIRE D'UNE CAMÉRA, ET DISPOSITIF ÉLECTRONIQUE ASSOCIÉ

(30) Priority: 04.01.2023 KR 20230001320
(43) Date of publication of application: 15.10.2025
(73) Proprietor: Samsung Electronics Co., Ltd., Suwon-si, Gyeonggi-do 16677 (KR)
(72) Inventor: KIM, Hyungrae, Suwon-si, Gyeonggi-do 16677 (KR)
(74) Representative: Appleyard Lees IP LLP
(86) International application number: PCT/KR2023/021874
(87) International publication number: WO 2024/147555

(56) References cited:
- JP-A- 2022 068 055
- KR-A- 20170 010 713
- KR-A- 20190 041 648
- KR-A- 20190 101 331
- KR-A- 20200 001 911
- US-A1- 2021 289 128
- US-A1- 2021 369 128
- US-A1- 2022 004 742
- RAPOSO AFONSO ET AL: "Camera-based Photoplethysmography (cbPPG) using smartphone rear and frontal cameras: an experimental study", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 7091 - 7094, XP034042150, DOI: 10.1109/EMBC46164.2021.9630847
- AMOGH GUDI ET AL: "Real-time Webcam Heart-Rate and Variability Estimation with Clean Ground Truth for Evaluation", ARXIV.ORG CORNELL UNIVERSITY LIBRARY, 201 OLIN LIBRARY CORNELL UNIVERSITY ITHACA, NY 14853, 31 December 2020 (2020-12-31), XP081849756, DOI: 10.3390/APP10238630

## Description

### Technical Field

The present disclosure relates to a method and an electronic device for controlling a display when obtaining biometric information by using a remote photoplethysmography (rPPG) device capable of obtaining biometric information through a camera.

### Background Art

Remote photoplethysmography (rPPG) refers to the use of light to measure a blood flow rate, which is a type of biometric information. When using rPPG technology, biometric information such as a heart rate or an oxygen saturation may be measured or calculated by using light at a body part where a blood vessel passes. As a heart pumps blood, blood flow rates of respective blood vessels distributed throughout a body are synchronized with a pulse and increase and decrease, and changes in the blood flow rate in a microvascular layer of a living tissue may be detected by using rPPG. For example, when using rPPG technology, widening or narrowing of blood vessels as a blood flow rate changes over time may be measured by using properties such as light absorbance and transmittance.

rPPG technology may be implemented relatively simply, and thus, has been employed in electronic devices such as mobile phones or home appliances and widely used to allow users to conveniently obtain information about their health. Various factors may affect the results of rPPG measurements using an electronic device. For example, measurement results obtained by using the rPPG technology may be affected by factors such as the user's skin tone or skin color, a motion artifact caused due to the user's motion, ambient light, or ambient temperature. The accuracy or reliability of such measurement results may decrease when affected by various factors.

Accordingly, to increase the accuracy of these measurement results, there is a demand for a technology for obtaining biometric information considering factors that may affect measurement, according to the type of an electronic device using rPPG technology. US 2021/0289128 A1 describes an image acquisition method that includes acquiring a first image based on a first photographing parameter, where the first image includes a first to-be-detected item, acquiring a second image based on a second photographing parameter, where the second image includes a second to-be-detected item, and determining, based on the first image and the second image, a detection result corresponding to the first to-be-detected item and a detection result corresponding to the second to-be-detected item. US 2022/0004742 A1 describes an ambient light parameter being acquired by detecting ambient light of a surveillance area.

Afonso et al "Camera-based Photoplethysmography (cbPPG) using smartphone rear and frontal cameras: an experimental study" describes monitoring of a PPG signal using a standard smartphone.

Gudi et al, "Real-time Webcam Heart-Rate and Variability Estimation with Clean Ground Truth for Evaluation", (31 December 2020) is further background art.

### Disclosure of Invention

### Solution to Problem

In an embodiment of the present disclosure, a method of obtaining biometric information through a camera may include sensing ambient brightness information, controlling the display to adjust a light output through the display based on the sensed ambient brightness information, obtaining, through the camera, an input image including a part of a body of a user, and obtaining biometric information of the user based on the input image.

In an embodiment of the present disclosure, an electronic device for obtaining biometric information through a camera may include the camera, a display, an illuminance sensor, a memory storing a program including at least one instruction, and at least one processor. The at least one processor may be configured to execute the at least one instruction stored in the memory to sense ambient brightness information through the illuminance sensor, control the display to adjust a light output through the display, based on the ambient brightness information, obtain, through the camera, an input image including a part of a body of a user, and obtain biometric information of the user based on the input image.

In an embodiment of the present disclosure, a method of controlling a display may include sensing ambient brightness information, and controlling the display to adjust a light output through the display based on the sensed ambient brightness information.

In an embodiment of the present disclosure, an electronic device for adjusting output light according to ambient brightness information may include a display, an illuminance sensor, a memory storing a program including at least one instruction, and at least one processor. The at least one processor may be configured to execute the at least one instruction stored in the memory to sense ambient brightness information through the illuminance sensor, and control the display to adjust a light output through the display, based on the ambient brightness information.

In an embodiment of the present disclosure, a method of controlling a display may include identifying distance information between the display and a subject, and controlling the display to adjust a light output through the display based on the identified distance information.

In an embodiment of the present disclosure, an electronic device for adjusting output light according to distance information between a display and a subject may include a camera, a display, a memory storing a program including at least one instruction, and at least one processor. The at least one processor may be configured to execute the at least one instruction stored in the memory to identify distance information between the display and the subject, and control the display to adjust a light output through the display based on the identified distance information.

In an embodiment of the present disclosure, a computer-readable recording medium may have recorded thereon a program for implementing a method of obtaining biometric information through a camera, the method including sensing ambient brightness information, controlling a display to adjust a light output through the display based on the sensed ambient brightness information, obtaining, through a camera, an input image including a part of a body of a user, and obtaining biometric information of the user based on the input image.

In an embodiment of the present disclosure, the computer-readable recording medium may have stored therein a program for executing, on a computer, at least one of embodiments of methods disclosed herein.

### Brief Description of Drawings

FIG. 1 is a diagram illustrating an electronic device for obtaining biometric information from an image captured through a camera, according to an embodiment of the present disclosure.
FIG. 2 is a diagram for describing operations, performed by an electronic device, of obtaining biometric information from an image captured through a camera, according to an embodiment of the present disclosure.
FIG. 3 is a block diagram illustrating a configuration of an electronic device for obtaining biometric information from an image captured through a camera, according to an embodiment of the present disclosure.
FIG. 4 is a diagram for describing an operation, performed by an electronic device, of controlling a display based on ambient brightness information and obtaining biometric information of a user, according to an embodiment of the present disclosure.
FIG. 5 is a diagram for describing an operation, performed by an electronic device, of controlling a display to adjust intensity of output light, according to an embodiment of the present disclosure.
FIG. 6 is a diagram for describing an operation, performed by an electronic device, of controlling a display to adjust output light to include auxiliary light, according to an embodiment of the present disclosure.
FIG. 7 is a diagram for describing an operation of partitioning a display screen of an electronic device into a plurality of areas, according to an embodiment of the present disclosure.
FIG. 8 is a diagram for describing an operation, performed by an electronic device, of controlling backlight according to the type of a display, according to an embodiment of the present disclosure.
FIG. 9 is a flowchart of a method of obtaining biometric information from an image captured through a camera, according to an embodiment of the present disclosure.
FIG. 10 is a block diagram of an electronic device according to an embodiment of the present disclosure.

### Mode for the Invention

Hereinafter, embodiments of the disclosure will be described in detail with reference to the accompanying drawings for those of skill in the art to be able to perform the disclosure without any difficulty. The present disclosure may, however, be embodied in many different forms and should not be construed as being limited to embodiments set forth herein. In addition, in order to clearly describe the present disclosure, portions that are not relevant to the description of the present disclosure are omitted, and similar reference numerals are assigned to similar elements throughout the specification.

Although the terms used herein for describing embodiments of the present disclosure are selected from among common terms that are currently widely used in consideration of their function in the present disclosure, the terms may be different according to an intention of those of ordinary skill in the art, a precedent, or the advent of new technology. Also, in particular cases, the terms are discretionally selected by the applicant of the present disclosure, in which case, the meaning of those terms will be described in detail in the corresponding embodiment. Therefore, the terms used herein are not merely designations of the terms, but the terms are defined based on the meaning of the terms and content throughout the present disclosure.

The singular expression may also include the plural meaning as long as it is not inconsistent with the context. All the terms used herein, including technical and scientific terms, may have the same meanings as those generally understood by those of skill in the art related to the specification.

Throughout the present disclosure, when a part "includes" an element, it is to be understood that the part may additionally include other elements rather than excluding other elements as long as there is no particular opposing recitation. In addition, as used herein, the terms such as "...er (or)", "... unit", "... module", etc., denote a unit that performs at least one function or operation, which may be implemented as hardware or software or a combination thereof.

Throughout the specification, when a part is referred to as being "connected to" another part, the part may be "directly connected to" the other part or be "electrically connected to" the other part through an intervening element. In addition, when an element is referred to as "including" a component, the element may additionally include other components rather than excluding other components as long as there is no particular opposing recitation.

As used herein, the expression "configured to" may be interchangeably used with, for example, "suitable for", "having the capacity to", "designed to", "adapted to", "made to", or "capable of", according to a situation. The expression "configured to" may not imply only "specially designed to" in a hardware manner. Instead, in a certain circumstance, the expression "a system configured to" may indicate the system "capable of" together with another device or components. For example, "a processor configured (or set) to perform A, B, and C" may imply a dedicated processor (e.g., an embedded processor) for performing a corresponding operation or a generic-purpose processor (e.g., central processing unit (CPU) or an application processor) capable of performing corresponding operations by executing one or more software programs stored in a memory.

Functions related to artificial intelligence according to the present disclosure are performed by a processor and a memory. The processor may include one or more processors. In this case, the one or more processors may be a general-purpose processor, such as a CPU, an application processor (AP), or a digital signal processor (DSP), a dedicated graphics processor such as a graphics processing unit (GPU) or a vision processing unit (VPU), or a dedicated artificial intelligence processor such as a neural processing unit (NPU). The one or more processors perform control to process input data according to predefined operation rules or an artificial intelligence model stored in the memory. In a case in which the one or more processors are dedicated artificial intelligence processors, the dedicated artificial intelligence processor may be designed with a hardware structure specialized for processing a particular artificial intelligence model.

The predefined operation rules or artificial intelligence model is generated via a training process. Here, being generated via a training process may mean that predefined operation rules or artificial intelligence model set to perform desired characteristics (or purposes), is generated by training a basic artificial intelligence model (or deep learning model) by using a learning algorithm that utilizes a large amount of training data. The training process may be performed by a device itself on which artificial intelligence according to the present disclosure is performed, or by a separate server and/or system. Examples of learning algorithms may include, for example, supervised learning, unsupervised learning, semi-supervised learning, and reinforcement learning, but are not limited thereto.

The artificial intelligence model (or deep learning model) may include a plurality of neural network layers. Each of the neural network layers has a plurality of weight values, and performs a neural network arithmetic operation via an arithmetic operation between an arithmetic operation result of a previous layer and the plurality of weight values. The plurality of weight values in each of the plurality of neural network layers may be optimized as a result of training the artificial intelligence model. For example, the plurality of weight values may be modified to reduce or minimize a loss or cost value obtained by the artificial intelligence model during a training process. The artificial neural network may include, for example, a deep neural network (DNN) and may include, for example, a convolutional neural network (CNN), a deep neural network (DNN), a recurrent neural network (RNN), a restricted Boltzmann machine (RBM), a deep belief network (DBN), a bidirectional recurrent deep neural network (BRDNN), a deep Q-network, or the like, but is not limited thereto.

In the present disclosure, 'video' or 'image sequence' may refer to a moving picture or a moving image. A video or an image sequence may include a series of still images in chronological order.

In the present disclosure, 'video frame' or 'image' may refer to a single still image that is output on a display. That is, in a video that creates a moving image by displaying consecutive scenes at short time intervals, 'video frame' or 'image' may refer to a single still image of each scene.

Hereinafter, the present disclosure will be described in detail with reference to the accompanying drawings.

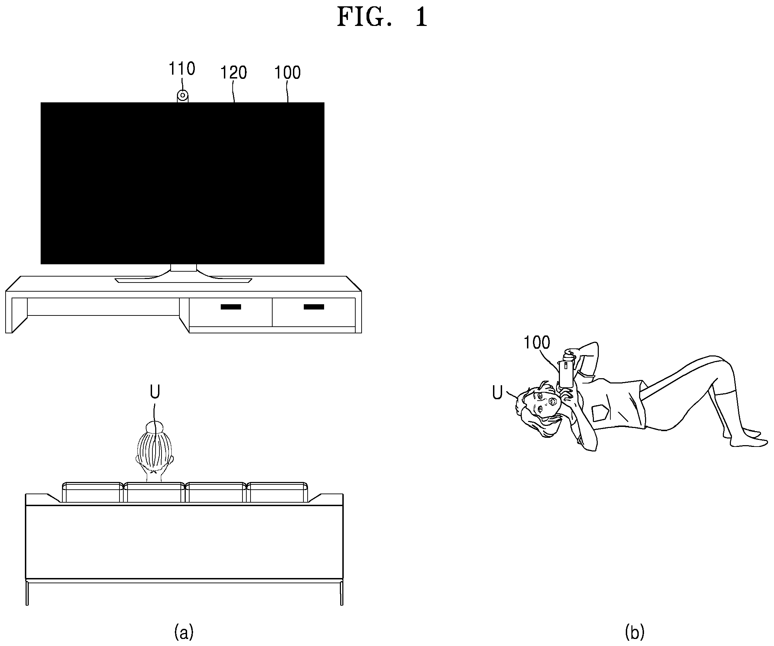

FIG. 1 is a diagram illustrating an electronic device 100 for obtaining biometric information from an image captured through a camera 110, according to an embodiment of the present disclosure.

In an embodiment of the present disclosure, the electronic device 100 is a device capable of remote photoplethysmography, and include the camera 110 and a display 120. The electronic device 100 may analyze a biometric signal (or biometric information) of a user U, such as a heart rate or a respiration rate, by analyzing an image obtained through the camera 110. For example, the camera 110 may include a red-green-blue (RGB) camera.

To measure biometric information of the user U (e.g., a heart rate, a respiration rate, a heart rate variability, or oxygen saturation), the electronic device 100 may use remote photoplethysmography (rPPG) technology. The electronic device 100 may calculate biometric information by measuring a degree of light absorption of hemoglobin in a blood vessel of the user U, based on a video obtained through the camera 110.

The electronic device 100 may be, for example, an electronic device including the display screen 120 and the camera 110, such as a television (TV), a tablet personal computer (PC), or a mobile phone. In addition, the electronic device 100 of the present disclosure is not limited to the above-described examples, and may include various types of devices including the camera 110 configured to capture an image for obtaining a biometric signal, and the display screen 120 that is to be controlled.

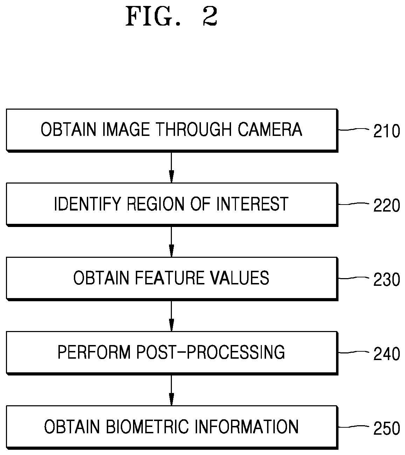

FIG. 2 is a diagram for describing operations, performed by an electronic device, of obtaining biometric information from an image captured through a camera, according to an embodiment of the present disclosure.

Referring to FIG. 2, in operation 210, the electronic device may obtain an image through the camera. The image may include an image corresponding to a part of a person's body, such as a face. Hereinafter, a user's face will be described as an example of an object photographed by the electronic device through the camera. In an embodiment, an object photographed through the camera may include various body parts of the body for obtaining biometric signals, such as a shoulder or an arm.

In operation 220, the electronic device may identify a region of interest (ROI) from the obtained image. The ROI may be, for example, a region including a face image corresponding to the user's face. The ROI may have a form of a block, a window, a bounding box, or a segmentation mask.

In an embodiment, in the operation of obtaining the ROI from the obtained image, a deep learning model trained to receive an image as an input, and output, as an ROI, an image region including a face region, may be used.

In an embodiment, in the operation of obtaining the ROI from the obtained image, a frame scanning operation or an edge analysis method may be used. In the frame scanning operation, a frame may be divided into a plurality of blocks of a certain size, and then the plurality of blocks may be scanned from an upper left to a lower right of the image to search for a region including a target image (e.g., a face image). Here, a size of the block may be preset. For example, through a processor, when it is determined that there are a plurality of blocks respectively including portions of the face because the block size is small, the scanning operation may be performed again after increasing the block size, whereas when it is determined that the pixel region including the face within a particular block is relatively small because the block size is large, the scanning operation may be performed again after decreasing the block size (within a corresponding block). For example, a size of a finally selected ROI may vary depending on a distance between the electronic device and the user's face. For example, when the user's face is close to the camera, the image portion corresponding to the user's face may be large, and thus, the block (window) size of the ROI may be large. For example, when the user's face is far from the camera, the block (window) size of the ROI may be set to be small.

In an embodiment, a position of an ROI detected in a moving image may fluctuate over time. When calculating feature values for biometric information measurement in a subsequent operation, difficulties may arise when the ROI fluctuates, and thus, a stabilization operation may be performed on the detected ROI by using an ROI stabilization module. In an embodiment, the ROI stabilization operation may be omitted.

In an embodiment, the ROI may be determined as a partial region of an image portion corresponding to the user's face. Although detection of rPPG signals from various parts of the user's body surface is possible, an area from which a feature value having a high significance may be obtained may be limited, depending on the biometric information to be measured. For example, when obtaining biometric information such as a heart rate, the ROI may be set to a region corresponding to a cheek or a forehead in the user's face may enable obtaining biometric information with higher accuracy. In an embodiment, the ROI may be determined as a cheek area or a forehead area, rather than an entire area of the user's face.

In operation 230, the electronic device may obtain a feature value to be used for obtaining biometric information within the determined ROI. The feature value may be feature value from which a biometric signal may be obtained. For example, because hemoglobin in blood absorbs green light well, a value or an average value of green channels in RGB channel data may be used as a feature value. For example, various pieces of information for calculating biometric information may be used as a feature value, such as a feature value effective for biometric information calculation that are obtained through RGB channel operations such as CHROME, or a feature value effective for biometric signal calculation that are obtained by using a DNN.

In operation 240, the electronic device may perform a post-processing operation on the obtained feature values. Because a feature value extracted from an image taken from a distance may contain noise due to various external factors (e.g., reflected light or camera noise), a post-processing operation may be performed on the obtained feature value to minimize an influence of noise on biometric information calculation. The post-processing operation may include, for example, applying a bandpass filter to the feature value to remove signal components outside a targeted biometric signal range.

For example, in a case in which the targeted biometric information is a heart rate, because a human heart rate is between approximately 48 bpm and 240 bpm, the post-processing operation may include applying, to the obtained feature value, a bandpass filter in which a minimum value of a passable frequency component is set to 48 bpm and a maximum value is set to 240 bpm. For example, in a case in which the targeted biometric information is a respiration rate, because a human respiration rate is between approximately 5 bpm and 30 bpm, the post-processing operation may include applying, to the obtained feature value, a bandpass filter in which the minimum value of the passable frequency component is set to 5 bpm and the maximum value is set to 30 bpm.

In an embodiment, the post-processing operation (240) for removing noise from the obtained feature value may be omitted. Alternatively, a DNN may be applied in the post-processing operation (240) instead of a bandpass filter.

In an embodiment, the operations of determining an ROI from an image obtained through a camera (220), extracting a feature value (230), and performing a post-processing operation for removing noise from the feature value (240) may be performed through one or more DNNs. For example, a series of operations of determining an ROI from an image obtained through a camera (220), extracting a feature value (230), and performing a post-processing operation for removing noise from the feature value (240) may be performed through a single DNN, or a DNN may be applied only to the post-processing operation (240) for removing noise from the feature value, only to the operation (230) of extracting a feature value from the ROI, or only to the operation (220) of determining the ROI from the image.

In operation 250, the electronic device may obtain biometric information by performing an operation, such as a fast Fourier transform (FFT), on the obtained feature value or the post-processed feature value. For example, frequencies of feature values may be analyzed by using an FFT operation, and a frequency having a strongest energy may be selected as a signal for representing biometric information. Thereafter, biometric information may be obtained based on an obtained FFT result value.

The operations performed in each of the operations shown in FIG. 2 may be modularized and may be implemented in a form of an application to be executed in the electronic device.

However, despite using the post-processing operation (240) to minimize the noise influence on the obtained biometric information, a measured biometric information value may be unstable depending on an environment in which the electronic device performs the operation of obtaining biometric information through a camera. For example, the measured biometric information value may be affected by a factor such as the distance between the electronic device and the user, ambient light, or a screen output by the electronic device through the display, and may be different from an actual biometric information value.

In an embodiment, in a case in which the electronic device includes a display, illuminance or color of a screen output through the display may affect the skin color of the user in the ROI to be recognized differently, and accordingly, the value of the obtained biometric information may have a difference from the actual biometric information value of the user.

For example, when the environment in which the electronic device is used includes sufficient ambient light (that is, the electronic device is used in a sufficiently bright environment), and the screen (light) output by the electronic device through the display is relatively dark, the influence, such as a change in the skin color of a captured body part of the user by light directly output by the electronic device, is small. However, when the environment in which the electronic device is used becomes darker, or the distance between the user and the display of the electronic device decreases, the skin color of the captured body part of the user may be recognized differently from the actual skin color due to light directly output by the electronic device, and biometric information obtained based on such a captured image may have an error compared to the actual biometric information of the user.

In an embodiment of the present disclosure, when an electronic device including a display performs an operation of obtaining biometric information through a camera (photoplethysmography or rPPG measurement), by adjusting the amount of light, color, or output position of a screen output through the display, etc., it is possible to minimize the influence of light output from the display on measured biometric information value and to improve the accuracy of biometric information measurement.

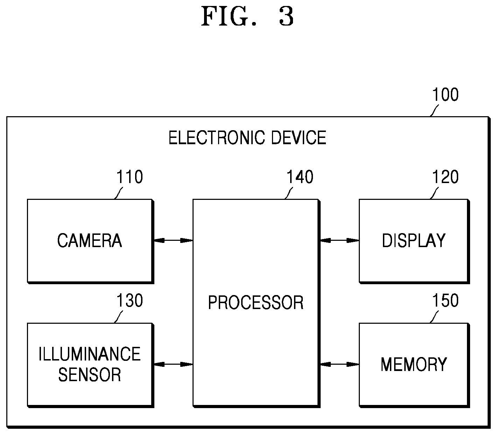

FIG. 3 is a block diagram illustrating a configuration of an electronic device for obtaining biometric information from an image captured through a camera, according to an embodiment of the present disclosure.

Referring to FIG. 3, the electronic device 100 may include the camera 110, the display 120, an illuminance sensor 130, a processor 140, and a memory 150. In addition, the electronic device 100 may be implemented by more components than those illustrated in FIG. 3.

The electronic device 100 may be a device for obtaining biometric information through the camera 110. For example, the electronic device 100 may perform the method described above with reference to FIG. 2. The electronic device 100 may be configured as, for example, at least one of a smart phone, a tablet PC, a mobile phone, a video phone, an e-book reader, a desktop PC, a laptop PC, a netbook computer, a TV, a home appliance, and other mobile or non-mobile computing devices. In addition, the electronic device 100 is not limited to the above-described examples, and the electronic device 100 may include various types of devices including the camera 110 capable of capturing a video for obtaining biometric information of a user, and the display 120.

The camera 110 may include a digital photographing device. The camera 110 may be a device for photographing light of various spectra. For example, the camera 110 may be a device for photographing light of various wavelengths, not limited to visible light. In an embodiment, the camera 110 may obtain a video, an image, or an image sequence including a part of the user's body. The electronic device 100 may obtain biometric information of the user through a change in the skin color of a part of the user's body included in an input video obtained through the camera 110.

The display 120 may be a device for displaying and outputting a visual image to an outside. For example, the display 120 may output a video or an image sequence to the outside. In an embodiment, the display 120 may include a panel. The display 120 may be configured as, for example, at least one of a liquid-crystal display, a digital mirror device, a liquid-crystal-on-silicon display device, a thin-film transistor-liquid-crystal display, an organic light-emitting diode (OLED), a micro-LED, a flexible display, a three-dimensional (3D) display, and an electrophoretic display.

The illuminance sensor 130 may be a device for sensing ambient brightness information of the electronic device 100 or the user of the electronic device 100. The illuminance sensor 130 may include an element having a photoelectric effect in which, when optical energy (light) is received, moving electrons are generated internally, changing the conductivity. The illuminance sensor 130 may determine a degree of brightness and darkness by using the conductivity, which varies depending on an amount of ambient light, and may obtain information about ambient brightness of the electronic device 100 or the user of the electronic device 100.

The memory 150 may store a program to be executed by the processor 140, which will be described below, to control the operation of the electronic device 100. The memory 150 may store a program including at least one instruction for controlling the operation of the electronic device 100. The memory 150 may store instructions and program code that are readable by the processor 140. In an embodiment, the processor 140 may be implemented to execute instructions or pieces of code of a program stored in the memory 150. The memory 150 may store data input to or output from the electronic device 100.

The memory 150 may include, for example, at least one of a flash memory-type storage medium, a hard disk-type storage medium, a multimedia card micro-type storage medium, a card-type memory (e.g., secure digital (SD) or extreme digital (XD) memory), a random-access memory (RAM), a static RAM (SRAM), a read-only memory (ROM), an electrically erasable programmable ROM (EEPROM), a programmable ROM (PROM), a magnetic memory, a magnetic disc, and an optical disc.

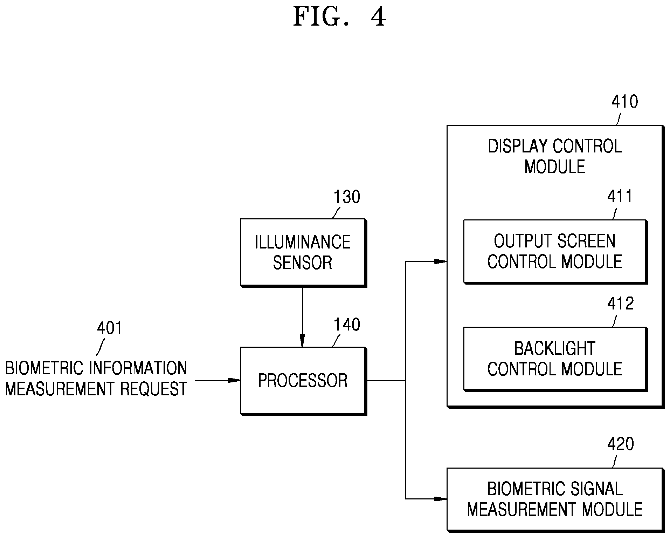

Programs stored in the memory 150 may be classified into a plurality of modules according to their functions. For example, the programs stored in the memory 150 may include a display control module and a biometric signal measurement module. The programs stored in the memory 150 will be described below in more detail with reference to FIG. 4.

The processor 140 may control an overall operation of the electronic device 100. The processor 140 may perform operations according to an embodiment of the present disclosure. For example, the processor 140 may execute programs stored in the memory 150 to control an overall operation of the camera 110, the display 120, the illuminance sensor 130, the memory 150, and the like.

The processor 140 may include a hardware component configured to perform arithmetic operations, logic operations, input/output operations, and signal processing. For example, the processor 140 may include at least one of a CPU, a microprocessor, a graphics processing unit, an application-specific integrated circuit (ASIC), a digital signal processor (DSP), a digital signal processing device (DSPD), a programmable logic device (PLD), and a field-programmable gate array (FPGA), but is not limited thereto.

The processor 140 may execute at least one instruction stored in the memory 150 to control the illuminance sensor 130 to sense ambient brightness information of the electronic device 100, and control the display 120, based on the sensed ambient brightness information, to adjust the output light. For example, the processor 140 may execute at least one instruction stored in the memory 150 to adjust brightness and color of an output image, or a waveform and a frequency of output light constituting the output image. The processor 140 may execute at least one instruction stored in the memory 150 to control the camera 110 to obtain an input such as an image including a part of the user's body, and obtain biometric information of the user based on the input image.

The operation, performed by the processor 140, of controlling the camera 110 to obtain an input such as an image including a part of the user's body may correspond to operation 210 of FIG. 2 described above. The operation, performed by the processor 140, of obtaining biometric information of the user based on the input image may correspond to operations 220 to 250 of FIG. 2 described above. The operation, performed by the processor 140, of controlling the display 120 to adjust the brightness, color, etc. of an output image will be described below in more detail with reference to FIGS. 4 to 9.

In an embodiment, the processor 140 may execute at least one instruction stored in the memory 150 to control the display 120 to adjust an intensity of output light.

For example, when a sensed ambient brightness value is less than a preset threshold value, the processor 140 may control the display 120 to decrease the intensity of the output light. That is, when a surrounding environment of the electronic device 100 is dark, the electronic device 100 may adjust a screen output through the display 120 to be dark in order to prevent biometric information from being distorted as a directly output image is reflected from a part of the user's body, such as the face.

For example, when the sensed ambient brightness value is less than the preset threshold value, the processor 140 may control the display 120 to adjust the output light to include auxiliary light. That is, when the surrounding environment of the electronic device 100 is dark, the electronic device 100 may display, on the screen of the display 120, an area to function as illumination to illuminate a part of the user's body, such as the face, in order to accurately obtain biometric information of the user.

In an embodiment, the processor 140 may execute at least one instruction stored in the memory 150 to control the display 120 to adjust the waveform of the output light. For example, the processor 140 may control the display 120 to change the waveform of the output light from a pulse wave to a continuous wave.

In an embodiment, the processor 140 may execute at least one instruction stored in the memory 150 to identify distance information between the display 120 and a part of the user's body included in an input image, and control the display 120, based on the identified distance information, to further adjust the output light. For example, distance information between the display 120 and a part of the user's body may be obtained through a separate distance sensor included in the electronic device 100, or may be calculated by using a time-of-flight (ToF) method based on the time it takes for light emitted from the display 120 to be reflected from the part of the user's body and then received by the camera 110.

For example, when an identified distance value is less than a preset threshold value, the processor 140 may control the display 120 to reduce the intensity of the output light. That is, when the screen (the display 120) of the electronic device 100 and a part of the user's body (e.g., the face) are close to each other, the electronic device 100 may adjust the screen output through the display 120 to be dark in order to prevent biometric information from being distorted as a directly output image is reflected from a part of the user's body.

For example, when the identified distance value is less than the preset threshold value, the processor 140 may control the display 120 to adjust the output light to include auxiliary light. That is, when the screen (the display 120) of the electronic device 100 and a part of the user's body (e.g., the face) are close to each other, the electronic device 100 may display, on the screen of the display 120, an area to function as illumination to illuminate the part of the user's body, in order to accurately obtain biometric information of the user.

In an embodiment, when a distance value between the display 120 and a part of the user's body included in an input image is less than a preset threshold value, the processor 140 may provide, through the display 120, the user with a guide for inducing the user to move to an appropriate position. For example, when it is determined that the user is too close to the display 120 such that the accuracy of obtained biometric information is likely to be degraded, the electronic device 100 may provide a guide such as "Please move back" such that the user may move to an appropriate distance from the electronic device 100. The guide may include, for example, a visual guide or an auditory guide. A guide for inducing the user to move may be provided, for example, when the output light cannot be adjusted.

In an embodiment, the processor 140 may execute at least one instruction stored in the memory 150 to partition the screen of the display 120 into a plurality of areas and adjust light output through each of the plurality of partitioned areas on a per-area basis.

For example, the processor 140 may adjust an output image displayed on a part of the display 120 to be dark, while displaying, on another part, a bright auxiliary light to function as illumination to illuminate a part of the user's body.

As such, according to an embodiment of the present disclosure, when obtaining an input such as an image including a part of the user's body through the camera 110, it is possible to, by controlling the display 120 to adjust the brightness, color, etc. of an output image, prevent a biometric signal measurement result from being distorted due to light output from the electronic device 100. In addition, by controlling the display 120 to adjust characteristics of the output image, such as brightness or color, a biometric signal may be measured well even in a dark environment.

FIG. 4 is a diagram for describing an operation, performed by an electronic device, of controlling a display based on ambient brightness information and obtaining biometric information of a user, according to an embodiment of the present disclosure.

Referring to FIG. 4, the electronic device may receive a biometric information measurement request 401 from a user or another electronic device. In an embodiment, the biometric information measurement request 401 may be received from the user or another electronic device through a biometric information measurement application or another application that requires biometric information.

The electronic device that has received the biometric information measurement request 401 may correspond to the electronic device 100 of FIG. 3 described above. In response to the biometric information measurement request 401, the processor 140 of the electronic device may control the illuminance sensor 130 to sense ambient brightness information. The illuminance sensor 130 may deliver the sensed ambient brightness information to the processor 140.

The processor 140 may execute at least one instruction stored in the memory to adjust output light to be output through the display, based on the ambient brightness information sensed by the illuminance sensor 130.

A program stored in the memory may be classified into a plurality of modules according to its functions. Referring to FIG. 4, the program stored in the memory may include a display control module 410 and a biometric signal measurement module 420.

The display control module 410 may be subdivided into one or more sub-modules according to a type of the display or a type of output light control. For example, in a case in which the display included in the electronic device is a self-luminous display such as an OLED display, the display control module 410 may include an output screen control module 411 for adjusting the intensity of an output screen displayed on the display screen. For example, in a case in which the display included in the electronic device is a non-self-luminous display such as an LCD, the display control module 410 may include a backlight control module 412 in addition to the output screen control module 411 for adjusting the intensity of an output screen displayed on the display screen.

The processor 140 may execute at least one instruction included in the display control module 410 to adjust output light based on ambient brightness information.

For example, the processor 140 may execute at least one instruction included in the output screen control module 411 of the display control module 410 to adjust the brightness (intensity), color, etc. of an output image. By using the output screen control module 411, the processor 140 may adjust the intensity of output light to decrease, or may adjust the intensity of output light to increase when the output light functions as illumination to illuminate a part of the user's body. By using the output screen control module 411, the processor 140 may adjust the color of the output light to white light, or may adjust the color of the output light to remove components that significantly affect rPPG signal measurement.

For example, the processor 140 may execute at least one instruction included in the backlight control module 412 of the display control module 410 to adjust the waveform, intensity, etc. of the output light. In a display including backlight, the waveform and intensity of the output light are determined based on the waveform and intensity of the backlight. By adjusting the intensity of the backlight by using the backlight control module 412, the processor 140 may adjust the intensity of the output light emitted based on the backlight. In addition, when the backlight has a waveform of a pulse wave, biometric information obtained through the camera may be distorted as the backlight is reflected on a part of the user's body, and thus, the processor 140 may change the waveform of the backlight from the pulse wave to a continuous wave.

Thereafter, the processor 140 may execute at least one instruction stored in the biometric signal measurement module 420 to obtain an input image including a part of the user's body through the camera and obtain biometric information of the user based on the input image. In an embodiment, the operation, performed by the processor 140, of obtaining biometric information of the user based on the input image through the biometric signal measurement module 420 may correspond to operations 210 to 250 shown in FIG. 2 described above.

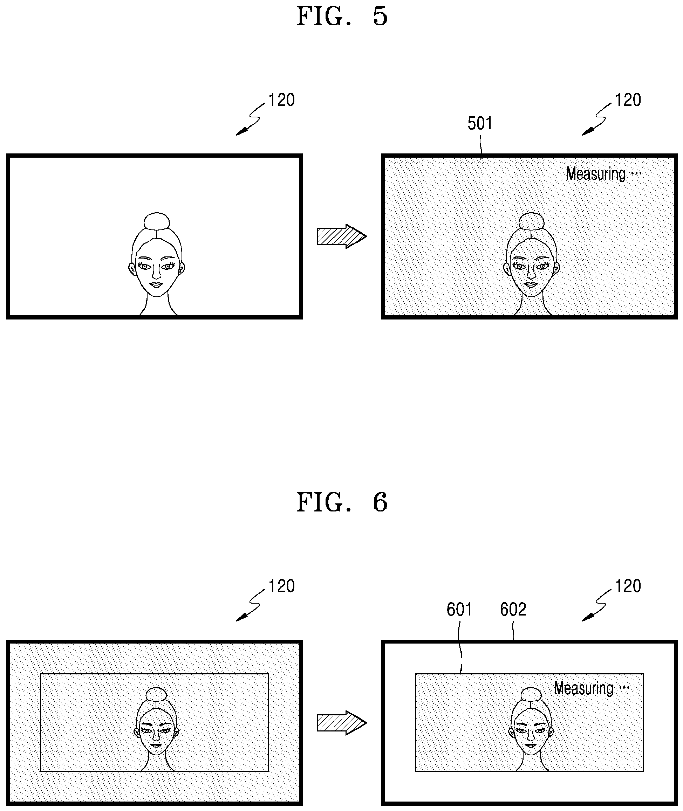

FIG. 5 is a diagram for describing an operation, performed by an electronic device, of controlling the display 120 to adjust the intensity of output light, according to an embodiment of the present disclosure.

In an embodiment, the electronic device may control the display 120 based on at least one of ambient brightness information or distance information between the display 120 and a part of the user's body included in an input image. The electronic device may control the display 120 to adjust the intensity of the output light.

Referring to FIG. 5, when a sensed ambient brightness value is less than a preset threshold value, or when the distance between the display 120 and a part of the user's body included in an input image is less than a preset threshold value, the electronic device may control the display 120 to adjust the intensity of the output light to decrease. That is, when the surrounding environment of the electronic device is dark or the screen (the display 120) of the electronic device and a part of the user's body (e.g., the face) are close to each other, the electronic device may adjust a screen 501 output through the display 120 to be darker in order to prevent biometric information from being distorted as a directly output image is reflected from a part of the user's body such as the face.

In an embodiment, the screen 501 output through the display 120 may include an execution screen of an application that obtains biometric information of the user. In an embodiment, the screen 501 output through the display 120 may further include an execution screen of another application to which multitasking may be applied, in addition to the execution screen of the application that obtains biometric information of the user.

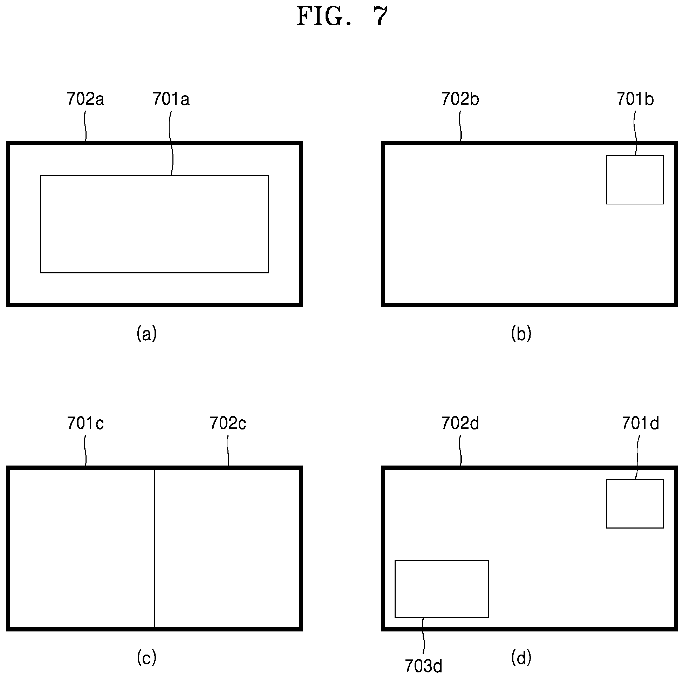

Referring to FIG. 5, brightness adjustment of the output light may be uniformly performed over the entire area of the display 120, but the present disclosure is not limited to this example embodiment, and as will be described below with reference to FIG. 7, the brightness of the output light may be individually adjusted for each of a plurality of partitioned areas of the screen of the display 120.

In an embodiment, when characteristics of the output light of the display 120, such as brightness or color, have been adjusted, the screen of the display 120 may include an indicator indicating that the characteristics of the output light have been adjusted. The indicator may prevent user confusion about the color or brightness of the output screen.

In an embodiment, when measurement of biometric information of the user is completed, the characteristics of the output light may be restored to a state before the adjustment.

FIG. 6 is a diagram for describing an operation, performed by an electronic device, of controlling the display 120 to adjust output light to include auxiliary light, according to an embodiment of the present disclosure.

In an embodiment, the electronic device may control the display 120 based on at least one of ambient brightness information or distance information between the display 120 and a part of the user's body included in an input image. The electronic device may control the display 120 to adjust characteristics of the output light, such as intensity or color.

Referring to FIG. 6, when a sensed ambient brightness value is less than a preset threshold value, or when the distance between the display 120 and a part of the user's body included in an input image is less than a preset threshold value, the electronic device may control the display 120 to adjust the output light to include auxiliary light. That is, when the surrounding environment of the electronic device is dark, or when the screen (the display 120) of the electronic device and a part of the user's body (e.g., the face) are close to each other, the electronic device 100 may display, on the screen of the display 120, an area 602 to function as illumination to illuminate a part of the user's body, such as the face, in order to accurately obtain biometric information of the user.

When the output light functions as illumination to illuminate a part of the user's body, the electronic device may adjust the intensity of the light that is output through the auxiliary light output area 602 to increase. In addition, the electronic device may adjust the color of the light that is output through the auxiliary light output area 602 to white or a particular color, or may adjust the color of the output light to remove components that significantly affect rPPG signal measurement.

In an embodiment, the screen that is output through the display 120 may include an execution screen 601 of an application that obtains biometric information of the user. The electronic device may adjust light that is output through the application execution screen 601. For example, when a sensed ambient brightness value is less than a preset threshold value, or when the distance between the display 120 and a part of the user's body included in an input image is less than a preset threshold value, the electronic device may adjust the intensity of light that is output through the application execution screen 601 to decrease, or may adjust the color of the output light to remove components that significantly affect rPPG signal measurement. That is, when the surrounding environment of the electronic device is dark or the screen (the display 120) of the electronic device and a part of the user's body (e.g., the face) are close to each other, the electronic device may adjust the execution screen 601 of the application to be dark in order to prevent biometric information from being distorted as a directly output image is reflected from a part of the user's body such as the face.

In this case, the adjusted output screen that is output through the display 120 may include, for example, the area of the application execution screen 601 where the brightness is adjusted to be low, and the auxiliary light output area 602 where the brightness is adjusted to be high.

In an embodiment, the screen that is output through the display 120 may further include an execution screen of another application to which multitasking may be applied, in addition to the execution screen 601 of the application that obtains biometric information of the user, and the auxiliary light output area 602. As will be described below with reference to FIG. 7, the brightness of the output light of the screen that is output through the display 120 may be adjusted individually for each of a plurality of partitioned areas of the screen of the display 120.

In an embodiment, when characteristics of the output light of the display 120, such as brightness or color, have been adjusted, the screen of the display 120 may include an indicator indicating that the characteristics of the output light have been adjusted. The indicator may prevent user confusion about the color or brightness of the output screen, and may be displayed on the execution screen 601 of the application or on the auxiliary light output area 602.

In an embodiment, when measurement of biometric information of the user is completed, the characteristics of the output light may be restored to the state before the adjustment.

FIG. 7 is a diagram for describing an operation of partitioning a display screen of an electronic device into a plurality of areas, according to an embodiment of the present disclosure.

In an embodiment, the electronic device may partition the display screen into a plurality of areas 701, 702, and 703, and adjust light that is output through each of the plurality of areas 701, 702, and 703 on a per-area basis.

Referring to (a) of FIG. 7, the display screen may include a first area 701a in a central portion, and a second area 702a surrounding the first area 701a. For example, an execution screen of a biometric information measurement application may be displayed on the first area 701a, and an execution screen of another application or an auxiliary light screen (an illumination screen) may be displayed on the second area 702a. In the embodiment of (a), when the execution screen of the other application is displayed on the second area 702a, the first area 701a where the execution screen of the biometric information measurement application is displayed may be implemented in a pop-up form.

Referring to (b) of FIG. 7, the display screen may include a first area 701b in an upper right portion, and a second area 702b in a portion other than the first area 701b. For example, an execution screen of a biometric information measurement application may be displayed on the first area 701b, and an execution screen of another application or an auxiliary light screen (an illumination screen) may be displayed on the second area 702b. In the embodiment of (b), when the execution screen of the other application is displayed on the second area 702b, the user may not stop a task currently being performed, even during biometric information measurement. In this case, even when biometric information measurement is not performed at a request from the user, biometric information may be measured without interfering with the user's task.

Referring to (c) of FIG. 7, the display screen may include a first area 701c on a left portion and a second area 702c on a right portion. For example, an execution screen of a biometric information measurement application may be displayed on the first area 701c and an execution screen of another application or an auxiliary light screen (an illumination screen) may be displayed on the second area 702c, or the execution screen of the biometric information measurement application may be displayed on the second area 702c and the execution screen of the other application or the auxiliary light screen (an illumination screen) may be displayed on the first area 701c. In the embodiment of (c), the user may monitor biometric information measurement without stopping a task currently being performed. Such a template may be effective during multitasking, or when biometric information measurement is performed in response to a request from the user but the user desires not to stop the task currently being performed.

Referring to (d) of FIG. 7, the display screen may include a first area 701d in an upper right portion, a third area 703d in a lower left portion, and a second area 702d in a portion other than the first area 701d and the third area 703d. For example, an execution screen of a biometric information measurement application or an execution screen of another application may be displayed on the first area 701d or the third area 703d, respectively, and an auxiliary light screen (an illumination screen) may be displayed on the second area 702d. The embodiment of (d) may be effective when multitasking is required in a dark environment, because the user may continue a task currently being performed without interruption even during biometric information measurement, and the output of auxiliary light is possible.

In addition, the electronic device may partition the display screen into four or more areas, and sizes and arrangements of the partitioned areas are not limited to the above-described embodiments. The display control method according to an embodiment of the present disclosure may be applied to various types of display screen templates.

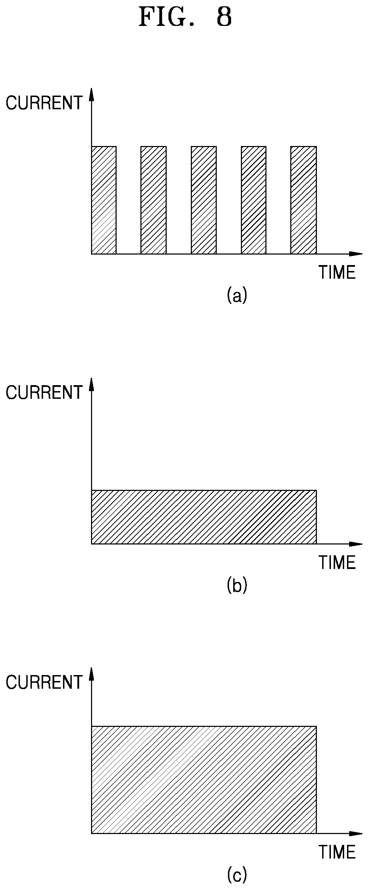

FIG. 8 is a diagram for describing an operation, performed by an electronic device, of controlling backlight according to the type of a display, according to an embodiment of the present disclosure.

In an embodiment, in a case in which the display included in the electronic device is a non-self-luminous display such as an LCD, the display may use backlight to output a screen. At least one of a current control method of controlling the backlight by using a current, or a pulse-width modulation (PWM) method of controlling the backlight by using a voltage may be applied. Among them, an LCD backlight to which the PWM method is applied may output light having a pulse waveform as illustrated in (a) of FIG. 8.

Referring to (a) of FIG. 8, a time interval between pulses of the LCD backlight is very short that it is imperceptible to a human, and a pulse frequency of the LCD backlight may be approximately 200 Hz or greater. In addition, light that is output through the display based on the LCD backlight with a pulse waveform also has a pulse waveform. When light having a pulse waveform is reflected from a part of the user's body, an input image obtained by photographing the body part may be distorted, and biometric information obtained based on the distorted input image may have an error from actual biometric information.

For example, when an electronic device including an LCD display measures the user's heart rate through a camera, and the pulse frequency of the LCD backlight is 1200 Hz, rPPG signals corresponding to frequencies corresponding to values obtained by dividing the pulse frequency by an integer, such as 30 Hz, 48 Hz, 50 Hz, 60 Hz, 80 Hz, 100 Hz, and 120 Hz, are constructively interfered with, and accordingly, a time point at which the constructive interference has occurred, rather than a time point at which the user's heart has actually beat, is more likely to be selected as data associated with the user's heart rate, and heart rate information of the user that is obtained in this way is different from the user's actual heart rate.

In an embodiment of the present disclosure, the electronic device may adjust at least one of the waveform or intensity of the backlight. When the display backlight has a waveform of a pulse wave as shown in (a) of FIG. 8, the electronic device may change the waveform of the backlight from the pulse wave to a continuous wave as shown in (b) or (c) of FIG. 8. When the waveform of the backlight is changed to a continuous wave, it is possible to prevent biometric information obtained through the camera from being distorted as the backlight is reflected on a part of the user's body.

In an embodiment, when a sensed ambient brightness value is less than a preset threshold value, or when the distance between a display operated through backlight and a part of the user's body included in an input image is less than a preset threshold value, the electronic device may change the waveform of the backlight to a continuous wave. That is, when the surrounding environment of the electronic device is dark, or when the screen (the display) of the electronic device and a part of the user's body (e.g., the face) are close to each other, the electronic device may change the backlight waveform of the display to a continuous wave to prevent interference between the backlight and biometric signals of the user, in order to prevent biometric information from being distorted as a directly output image is reflected from a part of the user's body such as the face.

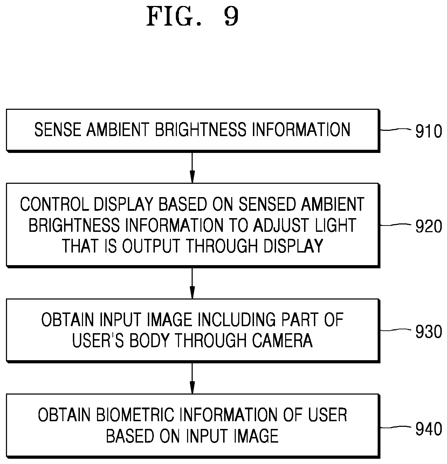

FIG. 9 is a flowchart of a method of obtaining biometric information from an image captured through a camera, according to an embodiment of the present disclosure.

In operation 910, the electronic device may sense ambient brightness information. In an embodiment, the electronic device may include an illuminance sensor, and may sense ambient brightness information of the electronic device through the illuminance sensor.

In operation 920, the electronic device may control the display based on the sensed ambient brightness information, to adjust light that is output through the display. In an embodiment, the electronic device may adjust the brightness and color of an output image, or the waveform and frequency of output light constituting the output image.

In an embodiment, the electronic device may control the display to adjust the intensity of the output light. For example, when a sensed ambient brightness value is less than a preset threshold value, the electronic device may control the display to adjust the intensity of the output light to decrease. That is, when the surrounding environment of the electronic device is dark, the electronic device may adjust a screen output through the display to be dark in order to prevent biometric information from being distorted as a directly output image is reflected from a part of the user's body, such as the face. For example, when the sensed ambient brightness value is less than the preset threshold value, the electronic device may control the display to adjust the output light to include auxiliary light. That is, when the surrounding environment of the electronic device is dark, the electronic device may display, on the screen of the display, an area to function as illumination to illuminate a part of the user's body, such as the face, in order to accurately obtain biometric information of the user.

In an embodiment, the electronic device may control the display to adjust the waveform of the output light. For example, the electronic device may control the display to change the waveform of the output light from a pulse wave to a continuous wave.

In an embodiment, the electronic device may identify distance information between the display and a part of the user's body included in an input image, and control the display, based on the identified distance information, to further adjust the output light. For example, distance information between the display and a part of the user's body may be obtained through a separate distance sensor included in the electronic device, or may be calculated by using a ToF method based on the time it takes for light emitted from the display to be reflected from the part of the user's body and then received by the camera.

For example, when an identified distance value is less than a preset threshold value, the electronic device may control the display to reduce the intensity of the output light. That is, when the screen (the display) of the electronic device and a part of the user's body (e.g., the face) are close to each other, the electronic device may adjust the screen output through the display to be dark in order to prevent biometric information from being distorted as a directly output image is reflected from a part of the user's body. For example, when the identified distance value is less than the preset threshold value, the electronic device may control the display to adjust the output light to include auxiliary light. That is, when the screen (the display) of the electronic device and a part of the user's body (e.g., the face) are close to each other, the electronic device may display, on the screen of the display, an area to function as illumination to illuminate the part of the user's body, in order to accurately obtain biometric information of the user.

In an embodiment, when a distance value between the display and a part of the user's body included in an input image is less than a preset threshold value, the electronic device may provide, through the display, the user with a guide for inducing the user to move to an appropriate position. For example, when it is determined that the user is too close to the display such that the accuracy of obtained biometric information is likely to be degraded, the electronic device may provide a guide such as "Please move back" such that the user may move to an appropriate distance from the electronic device. The guide may include, for example, a visual guide or an auditory guide. A guide for inducing the user to move may be provided, for example, when the output light cannot be adjusted.

In an embodiment, the electronic device may partition the display screen into a plurality of areas, and adjust light that is output through each of the plurality of areas on a per-area basis. For example, the electronic device may adjust an output image displayed on a part of the display to be dark, while displaying, on another part, a bright auxiliary light to function as illumination to illuminate a part of the user's body.

In operation 930, the electronic device may obtain an input image including a part of the user's body, through a camera. The operation, performed by the electronic device, of obtaining the input image including a part of the user's body through the camera may correspond to operation 210 of FIG. 2 described above.

In operation 940, the electronic device may obtain biometric information of the user based on the input image. The operation, performed by the electronic device, of obtaining biometric information of the user based on the input image may correspond to operations 220 to 250 of FIG. 2 described above.

As such, according to an embodiment of the present disclosure, when obtaining an input such as an image including a part of the user's body through the camera, it is possible to, by controlling the display to adjust characteristics of an output image, such as brightness or color, prevent a biometric signal measurement result from being distorted due to light output from the electronic device. In addition, by controlling the display to adjust the brightness, color, etc. of the output image, a biometric signal may be measured well even in a dark environment.

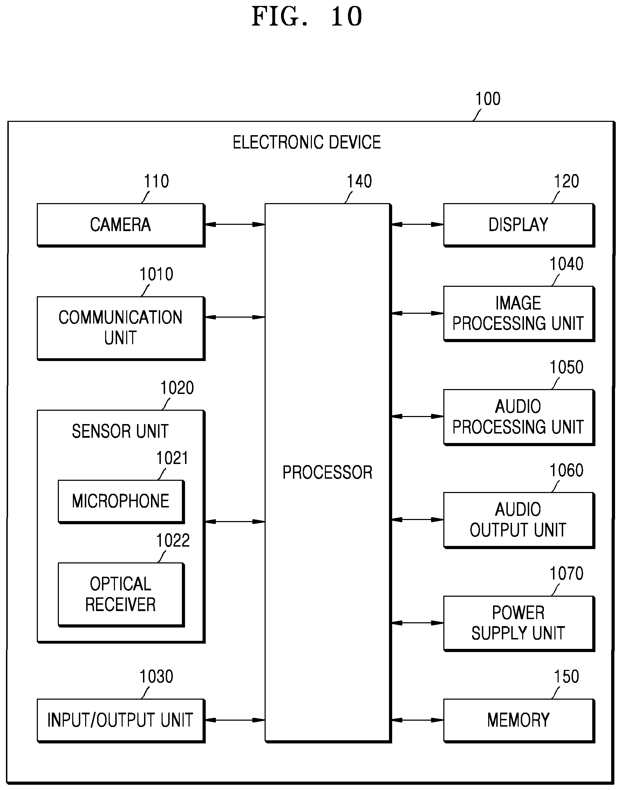

FIG. 10 is a block diagram of the electronic device 100 according to an embodiment of the present disclosure.

The electronic device 100 of FIG. 10 may be an embodiment of the electronic device 100 described above with reference to FIGS. 3 and 4. For example, the electronic device 100 of FIG. 10 may be an electronic device such as a smart TV or a mobile phone.

Referring to FIG. 10, the electronic device 100 may include the camera 110, the display 120, the processor 140, the memory 150, a communication unit 1010, a sensor unit 1020, an input/output unit 1030, an image processing unit 1040, an audio processing unit 1050, an audio output unit 1060, and a power supply unit 1070.

The camera 110 of FIG. 10 may correspond to the camera 110 of FIG. 3, the display 120 of FIG. 10 may correspond to the display 120 of FIG. 3, the processor 140 of FIG. 10 may correspond to the processor 140 of FIG. 3 or 4, and the memory 150 of FIG. 10 may correspond to the memory 150 of FIG. 3 or 4. Thus, redundant descriptions provided above will be omitted.

The communication unit 1010 according to an embodiment may include a Wi-Fi module, a Bluetooth module, an infrared communication module, a wireless communication module, a local area network (LAN) module, an Ethernet module, a wired communication module, and the like. Here, each communication module may be implemented as at least one hardware chip.

The Wi-Fi module and the Bluetooth module may perform communication by using a Wi-Fi scheme and a Bluetooth scheme, respectively. When the Wi-Fi module or the Bluetooth module is used, various pieces of connection information, such as a service set identifier (SSID) or a session key, may be first transmitted and received, and various pieces of information may be then transmitted and received after a communication connection is established by using the connection information. The wireless communication module may include at least one communication chip configured to perform communication according to various wireless communication standards, such as Zigbee, 3^{rd} Generation (3G), 3^{rd} Generation Partnership Project (3GPP), Long-Term Evolution (LTE), LTE Advanced (LTE-A), 4^{th} Generation (4G), 5^{th} Generation (5G), or the like.

The communication unit 1010 according to an embodiment may receive a user input from an external device.

The sensor unit 1020 may sense a user signal around the electronic device 100, and may include at least one of a microphone 1021 and an optical receiver 1022.

The microphone 1021 may receive a voice uttered by the user. The microphone 1021 may convert the received voice into an electrical signal and output the electrical signal to the processor 140. The microphone 1021 may perform various denoising algorithms for removing noise occurring when an external audio signal is received.

The camera 110 may obtain a still image or an image frame of a moving image or the like. An image captured through an image sensor may be processed by the processor 140 or a separate image processing unit.

An image frame processed by the camera 110 may be stored in the memory 150 or transmitted to the outside through the communication unit 1010. Two or more cameras 110 may be provided depending on the configuration of the electronic device 100.

The optical receiver 1022 may receive an optical signal (including a control signal) from an external remote control device. The optical receiver 1022 may receive, from a remote control device (not shown), an optical signal corresponding to a user input (e.g., a touch, a push, a touch gesture, a voice, or a motion). A control signal may be extracted from the received optical signal, under control of the processor 140. For example, the optical receiver 1022 may receive a control signal corresponding to a channel up/down button for channel switching, from a remote control device.

The sensor unit 1020 of FIG. 10 is illustrated as including the microphone 1021 and the optical receiver 1022, but is not limited thereto, and may include at least one of a magnetic sensor, an acceleration sensor, a temperature/humidity sensor, an infrared sensor, a gyroscope sensor, a position sensor (e.g., a global positioning system (GPS)), a barometric sensor, a proximity sensor, a red-green-blue (RGB) sensor, an illuminance sensor, a radar sensor, a light detection and ranging (LiDAR) sensor, and a Wi-Fi signal receiving unit, but is not limited thereto. Functions of the sensors may be intuitively inferred by those of skill in the art from their names, and thus, detailed descriptions thereof will be omitted.

The sensor unit 1020 of FIG. 10 is illustrated as being provided in the electronic device 100 itself, but is not limited thereto, and may be provided in a control device, which is a device that is positioned independently of the electronic device 100 and communicates with the electronic device 100, such as a remote controller.

In a case in which the sensor unit 1020 is provided in the control device of the electronic device 100, the control device may digitize information detected by the sensor unit 1020 and transmit the digitized information to the electronic device 100. The control device may communicate with the electronic device 100 by using short-range communication such as infrared, Wi-Fi, or Bluetooth.

The input/output unit 1030 may receive a video (e.g., a moving image), an audio (e.g., a voice or music), and additional information (e.g., an electronic program guide (EPG)) from the outside of the electronic device 100, under control of the processor 140. The input/output unit 1030 may include any one of a high-definition multimedia interface (HDMI) port, a mobile high-definition link (MHL) port, a universal serial bus (USB) port, a display port (DP), a Thunderbolt port, a video graphics array (VGA) port, an RGB port, a D-subminiature (D-SUB) port, a digital visual interface (DVI) port, a component jack, and a PC port.

The image processing unit 1040 may process image data received by the electronic device 100. The image processing unit 1040 may perform various image processing operations, such as decoding, scaling, noise filtering, frame rate conversion, or resolution conversion, on the image data.

The display 120 may convert an image signal, a data signal, an on-screen display (OSD) signal, a control signal, or the like, which has been processed by the processor 140, to generate a driving signal. The display 120 may be implemented as a plasma display panel (PDP), an LCD, an OLED, a flexible display, or a 3D display. In addition, the display 120 may be configured as a touch screen to be used as both an output device and an input device.

The display 120 may output various pieces of content input through the communication unit or the input/output unit 1030, or output an image stored in the memory 150. In addition, the display 120 may output information input by the user through the input/output unit 1030, on a screen.

The display 120 may include a display panel. The display panel may be an LCD panel or a panel including various light-emitting elements such as an LED, an OLED, or a cold cathode fluorescent lamp (CCFL). In addition, the display panel may include not only a flat display device but also a curved display device, which is a screen having a curvature or a flexible display device capable of adjusting a curvature. The display panel may be a 3D display or an electrophoretic display.

An output resolution of the display panel may include, for example, high definition (HD), full HD, ultra HD, or a higher resolution than ultra HD.

The audio processing unit 1050 may process audio data. The audio processing unit 1050 may perform various processing operations, such as decoding, amplification, or noise filtering, on the audio data. In addition, the audio processing unit 1050 may include a plurality of audio processing modules configured to process an audio corresponding to a plurality of pieces of content.

The audio output unit 1060 may output an audio included in a broadcast signal, under control of the processor 140. The audio output unit 1060 may output an audio (e.g., a voice or a sound) input through the communication unit 1010 or the input/output unit 1030. In addition, the audio output unit 1060 may output an audio stored in the memory 150 under control of the processor 140. The audio output unit 1060 may include at least one of a speaker or a headphone output port.

The power supply unit 1070 may supply power input from an external power source, to the components inside the electronic device 100 under control of the processor 140. In addition, the power supply unit 1070 may supply power output from one or more batteries in the electronic device 100, to internal components, under control of the processor 140.

The memory 150 may store various pieces of data, programs, or applications for driving and controlling the electronic device 100 under control of the processor 140. The memory 150 may include a broadcast receiving module, a channel control module, a volume control module, a communication control module, a speech recognition module, a motion recognition module, an optical receiving module, a display control module, an audio control module, an external input control module, a power control module, a power control module of an external device connected in a wireless manner (e.g., Bluetooth), a speech database (DB), or a motion DB, which is not illustrated. The modules and the DBs of the memory 150, which are not illustrated, may be implemented in the form of software for the electronic device 100 to perform a function of controlling broadcast reception, a channel control function, a volume control function, a communication control function, a speech recognition function, a motion recognition function, a light reception control function, a display control function, an audio control function, an external input control function, a power control function, or a power control function of an external device connected in a wireless manner (e.g., Bluetooth). The processor 140 may perform each function by using the software stored in the memory 150.

The block diagram of the electronic device 100 illustrated in FIG. 10 is a block diagram for an embodiment. Each of the components illustrated in the block diagram may be integrated, added, or omitted according to the specification of the electronic device 100 actually implemented. That is, two or more components may be integrated into one component, or one component may be divided into two or more components, as necessary. In addition, a function performed by each block is for describing embodiments, and its detailed operation or device does not limit the scope of the present disclosure.

Various embodiments may be implemented or supported by one or more computer programs, which may be produced from computer-readable program code and stored in a computer-readable medium. In the present disclosure, the terms "application" and "program" refer to one or more computer programs, software components, instruction sets, procedures, functions, objects, classes, instances, relevant data, which are suitable for an implementation in computer-readable program code, or a part thereof. The term "computer-readable program code" may include various types of computer code including source code, object code, and executable code. The term "computer-readable medium" may include various types of media that is accessible by a computer, such as a ROM, a RAM, a hard disk drive (HDD), a compact disc (CD), a digital video disc (DVD), or various types of a memory.

In addition, a machine-readable storage medium may be provided in the form of a non-transitory storage medium. Here, the term 'non-transitory storage medium' refers to a tangible device, and may exclude wired, wireless, optical, or other communication links that transmit temporary electrical or other signals. In addition, the term 'non-transitory storage medium' does not distinguish between a case in which data is stored in a storage medium semi-permanently and a case in which data is stored temporarily. For example, the non-transitory storage medium may include a buffer in which data is temporarily stored. The computer-readable medium may be any available medium that is accessible by a computer, and may include a volatile or non-volatile medium and a removable or non-removable medium. The computer-readable media includes media in which data may be permanently stored and media in which data may be stored and overwritten later, such as a rewritable optical disc or an erasable memory device.

Embodiments disclosed herein may be implemented by a software (S/W) program including instructions stored in a computer-readable storage medium. The computer is a device capable of invoking instructions stored in a storage medium and performing operations according to embodiments disclosed herein, and may include an electronic device according to embodiments disclosed herein.

According to an embodiment, methods according to various embodiments disclosed herein may be included in a computer program product and then provided. The computer program product may include an S/W program and a computer-readable recording medium storing the S/W program. For example, the computer program product may include a product in the form of an S/W program electronically distributed (e.g., a downloadable application) through a manufacturer of a device or an electronic market. For electronic distribution, at least part of the S/W program may be stored in a storage medium or temporarily generated. In this case, the storage medium may be a storage medium of a server of the manufacturer or a server of the electronic market, or a relay server that temporarily stores the S/W program.

The computer program product may include a storage medium of a server or a storage medium of a device, in a system consisting of the server and the device. Alternatively, when there is a third device (e.g., a smart phone) communicatively connected to the server or the device, the computer program product may include a storage medium of the third device. Alternatively, the computer program product may include an S/W program itself that is transmitted from the server to the device or the third device, or transmitted from the third device to the device.

In this case, any one of the server, the device, and the third device may execute the computer program product and perform the method according to embodiments disclosed herein. Alternatively, two or more of the server, the device, and the third device may execute the computer program product and perform the method according to embodiments disclosed herein in a distributed manner.

For example, the server (e.g., a cloud server or an artificial intelligence server) may execute the computer program product stored in the server, and may control the device communicatively connected to the server to perform the method according to embodiments disclosed herein.

As another example, the third device may execute the computer program product to control a device communicatively connected to the third device to perform the method according to an embodiment disclosed herein. When the third device executes the computer program product, the third device may download the computer program product from the server, and execute the downloaded computer program product. Alternatively, the third device may execute the computer program product provided in a preloaded state, and perform the method according to embodiments disclosed herein.

An embodiment of the present disclosure may provide an electronic device and a method for increasing the accuracy of obtaining biometric information and stabilizing performance by controlling a display when obtaining biometric information through a camera in an rPPG device.

In an embodiment of the present disclosure, a method of obtaining biometric information through a camera may include sensing ambient brightness information, adjusting light output through the display by controlling the display based on the sensed ambient brightness information, obtaining, through the camera, an input image including a part of a user's body, and obtaining biometric information of the user based on the input image.

The adjusting of the output light by controlling the display may include adjusting an intensity of the output light.

The adjusting of the output light by controlling the display may include, based on the sensed ambient brightness information being less than a preset threshold value, adjusting the intensity of the output light to decrease by controlling the display.

The adjusting of the output light by controlling the display may include, based on the sensed ambient brightness information being less than the preset threshold value, adjusting the output light to include auxiliary light by controlling the display.

The adjusting of the output light by controlling the display may include adjusting a waveform of the output light.

The adjusting of the output light by controlling the display may include changing the waveform of the output light from a pulse wave to a continuous wave.

The method may further include identifying distance information between the display and the part of the user's body included in the input image, and adjusting the output light by controlling the display based on the identified distance information.

The adjusting of the output light by controlling the display may include, based on the identified distance information being less than a preset threshold value, adjusting the intensity of the output light to decrease by controlling the display.

The method may further include, based on the identified distance information being less than the preset threshold value, providing the user through the display with a guide to induce the user to move to an appropriate position.

The adjusting of the output light by controlling the display may include partitioning a screen of the display into a plurality of areas, and adjusting the light output through each of the plurality of areas on a per-area basis.

In an embodiment of the present disclosure, an electronic device for obtaining biometric information through a camera may include the camera, a display, an illuminance sensor, a memory storing a program including at least one instruction, and at least one processor. The at least one processor may be configured to execute the at least one instruction stored in the memory to sense ambient brightness information through the illuminance sensor, adjust light output through the display by controlling the display based on the sensed ambient brightness information, obtain, through the camera, an input image including a part of a user's body, and obtain biometric information of the user based on the input image.

The at least one processor may be further configured to execute the at least one instruction stored in the memory to adjust an intensity of the output light by controlling the display.

The at least one processor may be further configured to execute the at least one instruction stored in the memory to, based on the sensed ambient brightness information being less than the preset threshold value, adjust the intensity of the output light to decrease by controlling the display.

The at least one processor may be further configured to execute the at least one instruction stored in the memory to, based on the sensed ambient brightness information being less than the preset threshold value, adjust the output light to include auxiliary light by controlling the display.

The at least one processor may be further configured to execute the at least one instruction stored in the memory to adjust a waveform of the output light by controlling the display.

The at least one processor may be further configured to execute the at least one instruction stored in the memory to change the waveform of the output light from a pulse wave to a continuous wave.

The at least one processor may be further configured to execute the at least one instruction stored in the memory to identify distance information between the display and the part of the user's body included in the input image, and adjust the output light by controlling the display based on the identified distance information.

The at least one processor may be further configured to execute the at least one instruction stored in the memory to, based on the sensed distance information being less than the preset threshold value, adjust the intensity of the output light to decrease by controlling the display.

The at least one processor may be further configured to execute the at least one instruction stored in the memory to partition a screen of the display into a plurality of areas, and adjust the light output through each of the plurality of areas on a per-area basis.

In an embodiment of the present disclosure, a method of controlling a display may include sensing ambient brightness information, and adjusting light output through the display by controlling the display based on the sensed ambient brightness information.

In an embodiment of the present disclosure, an electronic device for adjusting output light according to ambient brightness information may include a display, an illuminance sensor, a memory storing a program including at least one instruction, and at least one processor. The at least one processor may be configured to execute the at least one instruction stored in the memory to sense ambient brightness information through the illuminance sensor, and adjust light output through the display by controlling the display based on the sensed ambient brightness information.

In an embodiment of the present disclosure, a method of controlling a display may include identifying distance information between the display and a subject, and adjusting light output through the display by controlling the display based on the identified distance information.

In an embodiment of the present disclosure, an electronic device for adjusting output light according to distance information between a display and a subject may include a camera, a display, a memory storing a program including at least one instruction, and at least one processor. The at least one processor may be configured to execute the at least one instruction stored in the memory to identify distance information between the display and the subject, and adjust light output through the display by controlling the display based on the identified distance information.

In an embodiment of the present disclosure, a computer-readable recording medium may have recorded thereon a program for implementing a method of obtaining biometric information through a camera, the method including sensing ambient brightness information, adjusting light output through a display by controlling the display based on the sensed ambient brightness information, obtaining, through a camera, an input image including a part of a user's body, and obtaining biometric information of the user based on the input image.

In an embodiment of the present disclosure, the computer-readable recording medium may have stored therein a program for executing, on a computer, at least one of embodiments of methods disclosed herein.

While the present disclosure have been particularly shown and described, it will be understood by one of ordinary skill in the art that various changes in form and details may be made therein without departing from the spirit and scope of the present disclosure. Therefore, it should be understood that the above-described embodiments are provided as examples in all respects and do not limit the scope of the present disclosure. For example, each element described in a single type may be executed in a distributed manner, and elements described distributed may also be executed in an integrated form.

The scope of the present disclosure is not defined by the detailed description of the present disclosure but by the following claims, and all modifications or alternatives derived from the scope and spirit of the claims and equivalents thereof fall within the scope of the present disclosure.

## Claims

1. A method of operating an electronic device (100) comprising a camera (110) and a display (120), the method comprising:
sensing ambient brightness information;
controlling a backlight of the display (120) based on the sensed ambient brightness information to prevent a distortion of a remote photoplethysmography (rPPG) signal caused by a light output from the display (120);
obtaining, through the camera (110), an input image comprising a part of a body of a user (U); and
obtaining the rPPG signal of the user (U) from the input image to determine biometric information.

2. The method of claim 1, wherein the controlling the backlight of the display (120) comprises adjusting an intensity of the output light.

3. The method of claim 2, wherein the controlling the backlight of the display (120) comprises, based on the sensed ambient brightness information being less than a preset threshold value, adjusting the intensity of the output light to decrease.

4. The method of any one of claims 2 or 3, wherein the controlling the backlight of the display (120) comprises, based on the sensed ambient brightness information being less than a preset threshold value, adjusting the output light to include auxiliary light.

5. The method of any one of claims 1 to 4, wherein the controlling the backlight of the display (120) comprises adjusting a waveform of the output light.

6. The method of claim 5, wherein the controlling the backlight of the display (120) comprises changing the waveform of the output light from a pulse wave to a continuous wave.

7. The method of any one of claims 1 to 6, further comprising:
identifying distance information between the display (120) and the part of the body of the user (U) included in the input image; and
controlling the backlight of the display (120) based on the identified distance information.

8. The method of claim 7, wherein the controlling the backlight of the display (120) comprises, based on the identified distance information being less than a preset threshold value, adjusting the intensity of the output light to decrease.

9. The method of any one of claims 7 or 8, further comprising, based on the identified distance information being less than a preset threshold value, providing the user (U) through the display (120) with a guide to induce the user (U) to move to an appropriate position.

10. The method of any one of claims 1 to 9, wherein the controlling the backlight of the display (120) comprises:
partitioning a screen of the display (120) into a plurality of areas; and
adjusting the light output through each of the plurality of areas on a per-area basis.

11. An electronic device (100) for obtaining biometric information through a camera (110), the electronic device (100) comprising:
the camera (110);
a display (120);
an illuminance sensor (130);
a memory (150) storing a program comprising at least one instruction; and
at least one processor (140) configured to execute the at least one instruction stored in the memory (150) to sense ambient brightness information through the illuminance sensor (130), control a backlight of the display (120) based on the sensed ambient brightness information to prevent a distortion of a remote photoplethysmography (rPPG) signal caused by a light output from the display (120), obtain, through the camera (110), an input image comprising a part of a body of a user (U), and obtain the rPPG signal of the user (U) from the input image to determine biometric information.

12. The electronic device (100) of claim 11, wherein the at least one processor (140) is further configured to execute the at least one instruction stored in the memory (150) to adjust an intensity of the output light.

13. The electronic device (100) of claim 12, wherein the at least one processor (140) is further configured to execute the at least one instruction stored in the memory (150) to, based on the sensed ambient brightness information being less than a preset threshold value, adjust the output light to include auxiliary light.

14. The electronic device (100) of any one of claims 11 to 13, wherein the at least one processor (140) is further configured to execute the at least one instruction stored in the memory (150) to adjust a waveform of the output light.

15. The electronic device (100) of any one of claims 11 to 14, wherein the at least one processor (140) is further configured to execute the at least one instruction stored in the memory (150) to identify distance information between the display (120) and the part of the body of the user included in the input image, and control the backlight of the display (120) based on the identified distance information.

## Patentansprüche

1. Verfahren zum Betreiben einer elektronischen Vorrichtung (100), die eine Kamera (110) und eine Anzeige (120) umfasst, wobei das Verfahren Folgendes umfasst:
Erfassen von Umgebungshelligkeitsinformationen;
Steuern einer Hintergrundbeleuchtung der Anzeige (120) basierend auf den erfassten Umgebungshelligkeitsinformationen, um eine Verzerrung eines Fernphotoplethysmographie-(rPPG-) Signals zu verhindern, die durch eine Lichtausgabe von der Anzeige (120) verursacht wird;
Gewinnen, über die Kamera (110), eines Eingabebilds, das einen Teil eines Körpers eines Benutzers (U) umfasst; und
Gewinnen des rPPG-Signals des Benutzers (U) aus dem Eingabebild, um biometrische Informationen zu bestimmen.

2. Verfahren nach Anspruch 1, wobei das Steuern der Hintergrundbeleuchtung der Anzeige (120) das Einstellen einer Intensität des Ausgangslichts umfasst.

3. Verfahren nach Anspruch 2, wobei das Steuern der Hintergrundbeleuchtung der Anzeige (120), basierend darauf, dass die erfassten Umgebungshelligkeitsinformationen geringer als ein voreingestellter Schwellenwert sind, das Einstellen der Intensität des Ausgangslichts derart, dass sie abnimmt, umfasst.

4. Verfahren nach einem der Ansprüche 2 oder 3, wobei das Steuern der Hintergrundbeleuchtung der Anzeige (120), basierend darauf, dass die erfassten Umgebungshelligkeitsinformationen geringer als ein voreingestellter Schwellenwert sind, das Einstellen des Ausgangslichts derart, dass es Hilfslicht beinhaltet, umfasst.

5. Verfahren nach einem der Ansprüche 1 bis 4, wobei das Steuern der Hintergrundbeleuchtung der Anzeige (120) das Einstellen einer Wellenform des Ausgangslichts umfasst.

6. Verfahren nach Anspruch 5, wobei das Steuern der Hintergrundbeleuchtung der Anzeige (120) das Ändern der Wellenform des Ausgangslichts von einer Pulswelle zu einer kontinuierlichen Welle umfasst.

7. Verfahren nach einem der Ansprüche 1 bis 6, ferner umfassend:
Identifizieren von Abstandsinformationen zwischen der Anzeige (120) und dem Teil des Körpers des Benutzers (U), der in dem Eingabebild beinhaltet ist; und
Steuern der Hintergrundbeleuchtung der Anzeige (120) basierend auf den identifizierten Abstandsinformationen.

8. Verfahren nach Anspruch 7, wobei das Steuern der Hintergrundbeleuchtung der Anzeige (120), basierend darauf, dass die identifizierten Abstandsinformationen geringer als ein voreingestellter Schwellenwert sind, das Einstellen der Intensität des Ausgangslichts derart, dass sie abnimmt, umfasst.

9. Verfahren nach einem der Ansprüche 7 oder 8, ferner umfassend, basierend darauf, dass die identifizierten Abstandsinformationen geringer als ein voreingestellter Schwellenwert sind, das Bereitstellen einer Führung für den Benutzer (U) über die Anzeige (120), um den Benutzer (U) zu veranlassen, sich zu einer geeigneten Position zu bewegen.

10. Verfahren nach einem der Ansprüche 1 bis 9, wobei das Steuern der Hintergrundbeleuchtung der Anzeige (120) Folgendes umfasst:
Aufteilen eines Bildschirms der Anzeige (120) in eine Vielzahl von Bereichen; und
Einstellen der Lichtausgabe über jeden der Vielzahl von Bereichen auf einer flächenbezogenen Basis.

11. Elektronische Vorrichtung (100) zur Gewinnung biometrischer Informationen über eine Kamera (110), wobei die elektronische Vorrichtung (100) Folgendes umfasst:
die Kamera (110);
eine Anzeige (120);
einen Beleuchtungsstärkesensor (130);
einen Speicher (150), der ein Programm speichert, das mindestens eine Anweisung umfasst; und
mindestens einen Prozessor (140), der dazu konfiguriert ist, die mindestens eine in dem Speicher (150) gespeicherte Anweisung auszuführen, um Umgebungshelligkeitsinformationen über den Beleuchtungsstärkesensor (130) zu erfassen, eine Hintergrundbeleuchtung der Anzeige (120) basierend auf den erfassten Umgebungshelligkeitsinformationen zu steuern, um eine Verzerrung eines Fernphotoplethysmographie- (rPPG-) Signals zu verhindern, die durch eine Lichtausgabe von der Anzeige (120) verursacht wird, über die Kamera (110) ein Eingabebild zu gewinnen, das einen Teil eines Körpers eines Benutzers (U) umfasst, und das rPPG-Signal des Benutzers (U) aus dem Eingabebild zu gewinnen, um biometrische Informationen zu bestimmen.

12. Elektronische Vorrichtung (100) nach Anspruch 11, wobei der mindestens eine Prozessor (140) ferner dazu konfiguriert ist, die mindestens eine in dem Speicher (150) gespeicherte Anweisung auszuführen, um eine Intensität des Ausgangslichts einzustellen.

13. Elektronische Vorrichtung (100) nach Anspruch 12, wobei der mindestens eine Prozessor (140) ferner dazu konfiguriert ist, die mindestens eine in dem Speicher (150) gespeicherte Anweisung auszuführen, um, basierend darauf, dass die erfassten Umgebungshelligkeitsinformationen geringer als ein voreingestellter Schwellenwert sind, das Ausgangslicht derart einzustellen, dass es Hilfslicht beinhaltet.

14. Elektronische Vorrichtung (100) nach einem der Ansprüche 11 bis 13, wobei der mindestens eine Prozessor (140) ferner dazu konfiguriert ist, die mindestens eine in dem Speicher (150) gespeicherte Anweisung auszuführen, um eine Wellenform des Ausgangslichts einzustellen.

15. Elektronische Vorrichtung (100) nach einem der Ansprüche 11 bis 14, wobei der mindestens eine Prozessor (140) ferner dazu konfiguriert ist, die mindestens eine in dem Speicher (150) gespeicherte Anweisung auszuführen, um Abstandsinformationen zwischen der Anzeige (120) und dem Teil des Körpers des Benutzers, der in dem Eingabebild beinhaltet ist, zu identifizieren, und die Hintergrundbeleuchtung der Anzeige (120) basierend auf den identifizierten Abstandsinformationen zu steuern.

## Revendications

1. Procédé de fonctionnement d'un dispositif électronique (100) comprenant une caméra (110) et un dispositif d'affichage (120), le procédé comprenant :
la détection d'informations de luminosité ambiante ;
la commande d'un rétroéclairage du dispositif d'affichage (120) sur la base des informations de luminosité ambiante détectées pour empêcher une distorsion d'un signal de photopléthysmographie à distance (rPPG) causée par une lumière émise en sortie par le dispositif d'affichage (120) ;
l'obtention, à travers la caméra (110), d'une image d'entrée comprenant une partie du corps d'un utilisateur (U) ; et
l'obtention du signal rPPG de l'utilisateur (U) à partir de l'image d'entrée pour déterminer des informations biométriques.

2. Procédé de la revendication 1, ladite commande du rétroéclairage du dispositif d'affichage (120) comprenant le réglage d'une intensité de la lumière de sortie.

3. Procédé de la revendication 2, ladite commande du rétroéclairage du dispositif d'affichage (120) comprenant, sur la base que les informations de luminosité ambiante détectées sont inférieures à une valeur seuil prédéfinie, le réglage de l'intensité de la lumière de sortie pour diminuer.

4. Procédé de l'une quelconque des revendications 2 ou 3, ladite commande du rétroéclairage du dispositif d'affichage (120) comprenant, sur la base des informations de luminosité ambiante détectées qui sont inférieures à une valeur seuil prédéfinie, le réglage de la lumière de sortie pour inclure une lumière auxiliaire.

5. Procédé de l'une quelconque des revendications 1 à 4, ladite commande du rétroéclairage du dispositif d'affichage (120) comprenant le réglage d'une forme d'onde de la lumière de sortie.

6. Procédé de la revendication 5, ladite commande du rétroéclairage du dispositif d'affichage (120) comprenant le changement de la forme d'onde de la lumière de sortie d'une onde pulsée en une onde continue.

7. Procédé de l'une quelconque des revendications 1 à 6, comprenant en outre :
l'identification d'informations de distance entre le dispositif d'affichage (120) et la partie du corps de l'utilisateur (U) comprise dans l'image d'entrée ; et
la commande du rétroéclairage du dispositif d'affichage (120) sur la base des informations de distance identifiées.

8. Procédé de la revendication 7, ladite commande du rétroéclairage du dispositif d'affichage (120) comprenant, sur la base que les informations de distance identifiées sont inférieures à une valeur seuil prédéfinie, le réglage de l'intensité de la lumière de sortie pour diminuer.

9. Procédé de l'une quelconque des revendications 7 ou 8, comprenant en outre, sur la base que les informations de distance identifiées sont inférieures à une valeur seuil prédéfinie, la fourniture à l'utilisateur (U) à travers le dispositif d'affichage (120) d'un guide pour inciter l'utilisateur (U) à se déplacer vers une position appropriée.

10. Procédé de l'une quelconque des revendications 1 à 9, ladite commande du rétroéclairage du dispositif d'affichage (120) comprenant :
la division d'un écran du dispositif d'affichage (120) en une pluralité de zones ; et
le réglage de la lumière émise en sortie à travers chacune de la pluralité de zones sur une base par zone.

11. Dispositif électronique (100) destiné à l'obtention d'informations biométriques par l'intermédiaire d'une caméra (110), le dispositif électronique (100) comprenant :
la caméra (110) ;
un dispositif d'affichage (120) ;
un capteur d'éclairement (130) ;
une mémoire (150) stockant un programme comprenant au moins une instruction ; et
au moins un processeur (140) configuré pour exécuter l'au moins une instruction stockée dans la mémoire (150) pour détecter des informations de luminosité ambiante par l'intermédiaire du capteur d'éclairement (130), commander un rétroéclairage du dispositif d'affichage (120) sur la base des informations de luminosité ambiante détectées pour empêcher une distorsion d'un signal de photopléthysmographie à distance (rPPG) causée par une lumière émise en sortie par le dispositif d'affichage (120), obtenir, par l'intermédiaire de la caméra (110), une image d'entrée comprenant une partie du corps d'un utilisateur (U), et obtenir le signal rPPG de l'utilisateur (U) à partir de l'image d'entrée pour déterminer des informations biométriques.

12. Dispositif électronique (100) de la revendication 11, ledit au moins un processeur (140) étant en outre configuré pour exécuter l'au moins une instruction stockée dans la mémoire (150) pour régler une intensité de la lumière de sortie.

13. Dispositif électronique (100) de la revendication 12, ledit au moins un processeur (140) étant en outre configuré pour exécuter l'au moins une instruction stockée dans la mémoire (150) pour, sur la base des informations de luminosité ambiante détectées qui sont inférieures à une valeur seuil prédéfinie, régler la lumière de sortie pour inclure une lumière auxiliaire.

14. Dispositif électronique (100) de l'une quelconque des revendications 11 à 13, ledit au moins un processeur (140) étant en outre configuré pour exécuter l'au moins une instruction stockée dans la mémoire (150) pour régler une forme d'onde de la lumière de sortie.

15. Dispositif électronique (100) de l'une quelconque des revendications 11 à 14, ledit au moins un processeur (140) étant en outre configuré pour exécuter l'au moins une instruction stockée dans la mémoire (150) pour identifier des informations de distance entre le dispositif d'affichage (120) et la partie du corps de l'utilisateur comprise dans l'image d'entrée, et commander le rétroéclairage du dispositif d'affichage (120) sur la base des informations de distance identifiées.
